(19) Europäisches Patentamt
European Patent Office
Office européen des brevets

(11) Publication number: 0 381 913 A1

# (12) EUROPEAN PATENT APPLICATION

(21) Application number: 89810934.3

(51) Int. Cl.[5]: C07C 259/10, C07C 309/69, C07C 311/48

(22) Date of filing: 11.12.89

The title of the invention has been amended (Guidelines for Examination in the EPO, A-III, 7.3).

(30) Priority: 14.12.88 GB 8829204

(43) Date of publication of application: 16.08.90 Bulletin 90/33

(84) Designated Contracting States: AT BE CH DE ES FR GB GR IT LI LU NL SE

(71) Applicant: SANDOZ AG
Lichtstrasse 35
CH-4002 Basel(CH)
(84) BE CH ES FR GB GR IT LI LU NL SE

Applicant: SANDOZ-PATENT-GMBH
Humboldtstrasse 3
D-7850 Lörrach(DE)
(84) DE

Applicant: SANDOZ-ERFINDUNGEN Verwaltungsgesellschaft m.b.H.
Brunner Strasse 59
A-1235 Wien(AT)
(84) AT

(72) Inventor: Grina, Jonas
Neumattstrasse 35
CH-4142 Münchenstein(CH)
Inventor: Ebner, Cuno
Siegmattstrasse 20
CH-4460 Gelterkinden(CH)
Inventor: Kirkpatrick, Joel Lee
363 Sharon Drive
Barrington, Ill. 60010(US)
Inventor: Steiger, Arthur
Angensteinerstrasse 55
CH-4144 Arlesheim(CH)
Inventor: Busteed, Roslynn
Ackerstrasse 55
CH-407 Basel(CH)

## (54) Benzohydroxamic-acid derivatives.

(57) Derivatives of benzohydroxamic acid having the formulae

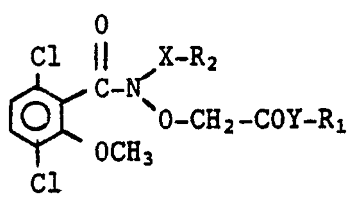

(I)

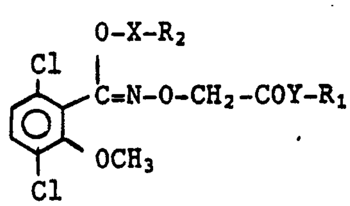

(II)

EP 0 381 913 A1

wherein X, Y, $R_1$, and $R_2$ are as herein disclosed, their use as herbicides, agricultural compositions containing the same, and processes for their manufacture.

## BENZOHYDROXAMIC DERIVATIVES

This application relates to derivatives of benzohydroxamic acid, their use as herbicides, to agricultural compositions containing the same, and to processes for their manufacture.

This application more particularly relates to compounds of the formulae (I) and (II):

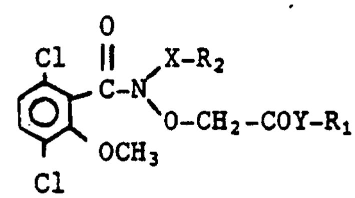

(I)

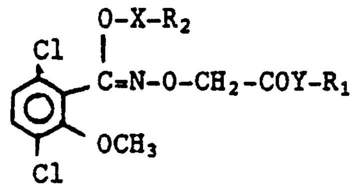

(II)

wherein

X is C(O) or $SO_2$;

Y is O or S;

$R_1$ is selected from the group consisting of H; $C_{1-12}$alkyl, unsubstituted or substituted by halogen; cyano-$C_{1-12}$alkyl; $C_{2-12}$alkenyl; $C_{2-12}$alkynyl; $C_{3-8}$cycloalkyl; $C_{2-5}$alkanoyl-$C_{1-5}$alkyl; $C_{2-5}$alkanoyl-oxy-$C_{1-5}$alkyl; di($C_{1-5}$alkoxycarbonyl-$C_{1-5}$alkyl)$C_{1-5}$alkyl; Ar-$(SO_2)_{n1}$-$(O)_{n2}$-$C_{1-5}$alkyl, wherein the $C_{1-5}$alkyl moiety is unsubstituted or substituted by CN;

$R_2$ is selected from the group consisting of H; $C_{1-12}$alkyl; $C_{2-12}$alkenyl; $C_{1-12}$alkoxy; $C_{2-12}$alkynyl; $C_{3-8}$cycloalkyl; benzyloxy; phenoxy; di($C_{1-5}$alkyl)amino; cyano; $C_{1-5}$alkoxy-carbonyl-$C_{1-5}$alkyl; and $Ar'$-$(O)_{n3}$-$C_{1-5}$alkyl;

Ar and $Ar'$ are independently phenyl or a five or six membered heteroaromatic ring containing one to three heteroatoms selected from the group consisting of oxygen and nitrogen, which phenyl or hetroaromatic ring may be unsubstituted or substituted by one or more substituents selected from the group consisting of halogen, $C_{1-5}$alkyl, $NO_2$, $C_{1-5}$alkoxy, CN, and $C_{1-5}$alkylthio;

n1, n2, and n3 are independently 0 or 1;

and salt forms thereof.

Where $R_1$ and/or $R_2$ are a $C_{1-12}$alkyl group, it is preferably a $C_{1-8}$, more preferably a $C_{1-4}$ alkyl group. Any alkyl group having three or more carbon atoms may be either branched or straight chain.

Where $R_1$ and/or $R_2$ are a $C_{1-12}$alkoxy group, it is preferably a $C_{1-8}$, more preferably a $C_{1-4}$ alkoxy group. Any alkoxy group having three or more carbon atoms may be either branched or straight chain.

Where $R_1$ and/or $R_2$ are a $C_{2-12}$alkenyl group, it is preferably a $C_{2-8}$, more preferably a $C_{2-5}$ alkenyl group. Any alkenyl group having three or more carbon atoms may be either branched or straight chain.

Where $R_1$ and/or $R_2$ are a $C_{2-12}$alkynyl group, it is preferably a $C_{2-8}$, more preferably a $C_{2-5}$ alkynyl group. Any alkynyl group having three or more carbon atoms may be either branched or straight chain.

Where $R_1$ and/or $R_2$ are a $C_{3-8}$cycloalkyl group, it is preferably a $C_{3-5}$ cycloalkyl group, more preferably cyclopropyl or cyclopentyl.

Where $R_1$ and/or $R_2$ contain an alkanoyl group having four or five carbon atoms, the alkanoyl group may be either branched or straight chain.

Where $R_1$ and/or $R_2$ contain halogen, it is preferably chlorine, bromine, fluorine, or iodine.

Where $R_1$ is halo-substituted $C_{1-12}$alkyl, it preferably comprises from one to four carbon atoms and from one to three halogen atoms.

Where $R_1$ is $C_{1-12}$cyanoalkyl, it is preferably $C_{1-5}$cyanoalkyl, e.g., $CH_2CH_2CN$.

Where Ar and/or $Ar'$ are substituted phenyl, they are preferably substituted by one to three substituents selected from the group consisting of halogen, $C_{1-4}$alkyl, $C_{1-4}$alkoxy, cyano and nitro.

Where Ar and/or Ar′ are a five or six membered heteroaromatic ring, they are preferably furyl. Where Ar and/or Ar′ are a substituted five or six membered heteroaromatic ring, they are preferably substituted by one or two substituents selected from the group consisting of $C_{1-4}$alkyl, halogen, and $C_{1-5}$alkylthio.

A preferred subgroup of compounds of formula (I) has one or more of the following features:

$R_1$ is $C_{1-5}$alkyl, unsubstituted or substituted by one to three halogen atoms; $C_{3-6}$cycloalkyl; $C_{1-5}$alkoxy-carbonyl-$C_{1-5}$alkyl; $Ar_1$-$C_{1-2}$alkyl, wherein $Ar_1$ is furyl or is phenyl unsubstituted or substituted by one or two substituents selected from the group consisting of halogen, nitro and $C_{1-4}$alkoxy; or is Ph-CH(CN), wherein Ph is phenyl;

$R_2$ is $C_{1-12}$alkyl; $C_{1-4}$alkoxy; $C_{3-6}$cycloalkyl; $C_{2-5}$alkenyl; benzyloxy; or phenyl, monosubstituted by $C_{1-4}$alkyl or halogen.

A preferred subgroup of the compounds of formula (II) has one or more of the following features:

X is C(O);

Y is O;

$R_1$ is $C_{1-4}$alkyl; $C_{3-6}$cycloalkyl; and

$R_2$ is $C_{1-4}$alkyl; $C_{2-4}$alkenyl; $C_{3-6}$cycloalkyl; or is phenyl mono-substituted by $C_{1-4}$alkyl or halogen.

The compounds of formulae (I) and (II) may be prepared by treating a compound of the formula (III)

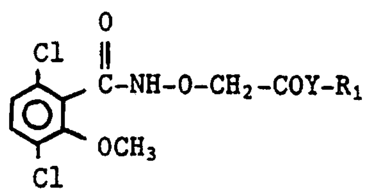
(III)

wherein Y and $R_1$ are as previously defined,

with an acyl halide or sulfonyl halide of the formula (IV)

$V-X-R_2$ (IV)

wherein V is halogen and $X-R_2$ are as defined above, in the presence of a base. Preferably, V is chlorine.

This process can be carried out analogous to processes known for the O- or N-acylation of amides. Suitable solvents include diethyl ether, tetrahydrofuran, dimethoxyethane, toluene, xylene, pyridine, dichloromethane, chloroform, dimethylformamide, dimethylsulfoxide, methyl-t-butyl ether, and mixtures thereof.

The temperature at which the reaction is carried out will depend largely on the choice of solvent, but it will normally be in the range of from -20° C to 100° C.

Compounds of formulae (III) and (IV) will react in the presence of a base to yield a mixture of compounds of formulae (I) and (II). The ratio of compound of formula (I) to compound of formula (II) in the mixture will depend, in part, upon the nature of the acyl halide and sulfonyl halide, and the reaction conditions. In general, non-polar solvents (e.g., xylene and toluene), and bulky halide reagents (e.g. benzyl chloride, i.e., reagents in which $R_2$ is large), tend to favor the production of compounds of formula (II). Conversely, polar solvents (e.g., pyridine and tetrahydrofuran) and halide reagents in which $R_2$ is small (e.g., acetyl chloride), tend to favor production of the compounds of formula (I).

A mixture of compounds of formulae (I) and (II) may be separated by known separation techniques, e.g., flash chromatography.

The compounds of formulae (I) and (II) may be converted into agriculturally acceptable salt form by known acid addition reactions where $R_1$ is H.

The compounds of formula (III) can be prepared by reacting a compound of formula (V)

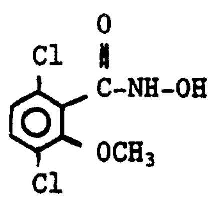
(V)

with a compound of the formula (VI)

$W-CH_2-\overset{O}{\overset{\parallel}{C}}-Y-R_1$ (VI)

wherein W is halogen, preferably chloro or bromo, and Y and $R_1$ are as defined above.

This reaction may be carried out at temperatures of from about 25°C to 150°C, preferably 60°C to 120°C in the presence of a base and in a solvent media. Preferred bases are the alkali metal hydroxides such as sodium hydroxide or potassium hydroxide. Preferred solvents are the lower alkanols such as methanol or ethanol or a mixture of water and a lower alkanol, e.g., water and ethanol. The desired product of formula (III) may be isolated and recovered by working up by established procedures.

Certain of the compounds of formula (III) are believed novel, and form a further aspect of this invention. Of particular interest are compounds of formula (IIIa)

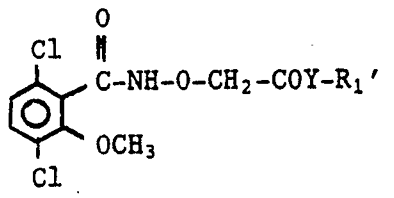

(IIIa)

in which

Y is O or S; and

$R_1'$ is $C_{1-5}$alkyl substituted by cyano; $C_{2-5}$alkynyl; $C_{2-5}$alkanoyl-$C_{1-5}$alkyl; $C_{2-5}$alkanoyl-oxy-$C_{1-5}$alkyl; $Ar_2$-$C_{1-5}$alkyl wherein the alkyl moiety is substituted by CN; or $Ar_2$-O-$C_{1-5}$alkyl; wherein $Ar_2$ is phenyl, unsubstituted or substituted by one or two substituents selected from halogen and $C_{1-4}$alkoxy.

Examples of such preferred compounds of formula IIIa include compounds 4.23, 4.24, 4.27, 4.29, 4.36, 4.38, and 4.39 from Table 4, set forth below.

Other preferred compounds of formula III are compounds 4.15, 4.32, 4.33, 4.37, and 4.40, taken from Table 4, set forth below; they are hereinafter, for convenience, designated Compounds IIIb.

Compounds of the formula (V) are prepared according to known procedures, for example, by adding the corresponding benzoyl chloride of the compound of formula (V) dropwise to a solution containing potassium carbonate and hydroxylamine hydrochloride in water and diethyl ether. The crude product may subsequently be washed with water and treated with HCl to yield the benzoylhydroxamic acid.

Insofar as the production of starting materials used in preparation of the compounds disclosed in this application are not particularly described, these compounds are either known or may be prpared from known materials by conventional methods (see, for example, EP A 0 255 800).

The compounds of the formulae (I), (II) and (IIIa) and Compounds (IIIb) (including the agriculturally acceptable salts thereof), hereinafter referred to as compounds of the invention, are useful because they control the growth of plants. By plants it is meant germinating seeds, merging seedlings and established vegetation including underground portions. In particular, the compounds are useful as herbicides as indicated by causing damage to both monocotyledoneous and dicotyledoneous plants in various standard evaluations for determining such effects. The herbicidal effects are exhibited both pre- and post-emergence the plants. Such herbicidal effects indicate that the compounds of the formulae (I), (II) and (IIIa), and Compounds IIIb are particularly of interest in combatting weeds (unwanted plants).

The compounds of the formulae (I), (II) and (IIIa), and Compounds IIIb are indicated mainly to be stronger acting against dicotyledoneous plants than monocotyledoneous plants. Relatively less toxicity towards crops than towards weeds is further indicated. Hence, the compounds are of particular interest as selective herbicides to combat weeds in a crop locus, particularly as locus of a monocotyledoneous crop such as, for example, corn (maize), oats, rice, wheat, sorghum and the like, especially corn.

The present invention therefore also provides a method of combatting weeds in a locus which comprises applying to the weeds or their locus a herbicidally effective amount of a compound of the invention. When selective action is desired in crop locus, the amount applied will be sufficient to combat weeds without substantially damaging the crop.

For general herbicidal as well as selective herbicidal use of the compounds of the invention, the particular amounts to be applied will vary depending upon recognized factors such as the compound employed, the plants primarily in the locus, the timing, mode and formulation in application, the various conditions of treatment such as soil and weather and the like. However, in general, satisfactory results in weed control are usually obtained upon application of the compounds of the invention at a rate in the range of from 0.05 to 10 kg/hectare, more usually 0.05 to 2 kg/hectare, and preferably 0.1 to 1 kg/hectare, the application being repeated as necessary. When used in crops, the application usually will not exceed about 5 kg/hectare, and is usually in the range of 0.1 to 2 kg/hectare.

Fcr practical use as herbicides, the compounds of the formulae (I), (II), and (IIIa) and Compounds IIIb may be and are preferably employed in herbicidal compositions comprising a herbicidal effective amount of the compound and an inert carier which is agriculturally acceptable in the sense of not, by reason of its presence, poisoning the agricultural environment including the immediate soil of application or any crops present therein or otherwise being unsafe for application. Such compositions of formulations may contain 0.01% to 99% by weight of active ingredient, from 0 to 20% by weight of agriculturally acceptable surfactants and 1 to 99.99% by weight of the inert carrier. Higher ratios of surfactant to active ingredient are sometimes desirable and are achieved by incorporation into the formulation or by tank mixing. Application forms of composition typically contain between 0.01 and 25% by weight of active ingredient, but lower or higher levels of active ingredient can, of course, be present depending on the intended use and the physical properties of the compound. Concentrate forms of composition intended to be diluted before use generally contain between 2 and 90%, preferably between 10 and 80% by weight of active ingredient.

Useful compositions or formulations of the compounds of the invention include dusts, granules, pellets, suspension concentrates, wettable powders, emulsifiable concentrates and the like. They are obtained by conventional manner, e.g. by mixing the compounds of the invention with the inert carrier. More specifically, liquid compositions are obtained by mixing the ingredients, fine solid compositions by blending and, usually grinding, suspensions by wet milling and granules and pellets by impregnating or coating (preformed) granular carriers with the active ingredient or by agglomeration techniques.

For example, dusts can be prepared by grinding and blending the active compound with a solid inert carrier such as talc, clay, silica and the like. Granular formulations can be prepared by impregnating the compound, usually dissolved in a suitable solvent, onto and into granulated carriers such as the attapulgites or the vermiculites, usually of a particle size range of from about 0.3 to 1.5 mm. Wettable powders, which can be dispersed in water or oil to any desired concentration of the active compound, can be prepared by incorporating wetting agents into concentrated dust compositions.

Alternatively, the compounds of the invention may be used in micro-encapsulated form.

Agriculturally acceptable additives may be employed in the herbicidal compositions to improve the performance of the active ingredient and to reduce foaming, caking and corrosion.

Surfactant as used herein means agriculturally acceptable material which imparts emulsifiability, spreading, wetting, dispersiblity or other surface-modifying properties properties. Examples of surfactants are sodium lignin sulphonate and lauryl sulphate.

Carriers as used herein mean a liquid or solid material used to dilute a concentrated material to a usable or desirable strength. For dusts or granules it can be e.g. talc, kaolin or diatomaeous earth, for liquid concentrate forms, a hydrocarbon such as xylene or an alcohol such as isopropanol; and for liquid application forms, eg. water or diesel oil.

The compositions of this application can also comprise other compounds having biological activity, e.g. compounds having similar or complementary herbicidal ativity or compounds having antidotal, fungicidal or insecticidal activity.

Typical herbicidal composition, according to this invention, are illustrated by the following Examples A, B and C in which the quantities are in parts by weight.

## EXAMPLE A

### Preparation of a Dust

10 Parts of a compound according to this invention and 90 parts of powdered talc are mixed in a mechanical grinder-blender and are ground until a homogeneous, free-flowing dust of the desired particle size is obtained. This dust is suitable for direct application to the site of the weed infestation.

## EXAMPLE B

### Preparation of Wettable Powder

25 Parts of a compound according to this invention are mixed and milled with 25 parts of synthetic fine silica, 2 parts of sodium lauryl sulphate, 3 parts of sodium ligninsulphonate and 45 parts of finely divided

kaolin until the mean particle size is about 5 micron. The resulting wettable powder is diluted with water before use to a spray liquor with the desired concentration.

## EXAMPLE C

Preparation of Emulsifiable Concentrate (EC)

13.37 Parts of a compound according to this invention are mixed in a beaker with 1.43 parts of Toximul 360A (a mixture of anionic and non-ionic surfactants containing largely anionic surfctants), 5.61 parts of Toximul 360A (a mixture of anionic and non-ionic surfactants containing largely non-ionic surfactants), 23.79 parts of dimethylformamide and 55.8 parts of Tenneco 500-100 (predominantly a mixture of alkylated aromatics such as xylene and ethylbenzene) until solution is effected. The resulting EC is diluted with water for use.

## FINAL COMPOUNDS

In the following examples, temperatures are in Celsius and "Ph" represents phenyl.

Example 1-1

Methyl N-acetyl-N-(3,6-dichloro-2methoxy)benzoylaminooxyacetate

To a solution of 3.1 g methyl N-(3,6-dichloro-2-methoxy)benzoylaminooxyacetate in 100 ml pyridine at -5° C, is added dropwise over 30 min, a solution of 2.3 ml acetyl chloride in 7.0 ml tetrahydrofuran. The resulting mixture is stirred for 1 hr at 0° C, then allowed to warm to ambient temperature and evaporated to dryness in vacuo. The residue is dissolved in ethyl acetate and washed four times with water. The organic phase is then dried over $MgSO_4$, filtered and evaporated in vacuo to yield 4.3 g crude product as an oil.

The crude product is purified by flash chromatography on a silica gel column with 9:1 dichloromethane:hexane. Fractions 2-8 are combined and evaporated to afford 2.68 of a colourless syrup that is consistent with the desired product by IR and $^1$H NMR. The $^1$H NMR spectrum, at 360MHz, shows line-broadening at room temperature that coalesces at 150° C to a single set of sharp resonances.

In an analogous manner, the following compounds of formula (I) set forth in Table 1 are made.

# TABLE 1

Compounds of the formula (I), wherein X is C(O) and Y is O, except compounds 1.78 and 1.83 wherein Y is S.

| Cmpd. | R1 | $R_2$ | Characteristic |
|---|---|---|---|
| 1.2 | $-CH_2(CH_2)_4CH_3$ | $-CH_3$ | $n^{20}D$ 1.5143 |
| 1.3 | $-CH_3$ | $-CH_2Cl$ | m.p. 102-103°C |
| 1.4 | $-CH_2$(2,4-diCl-Ph) | $-CH_3$ | thick syrup |
| 1.5 | $-CH_2$(2,4-diCl-Ph) | $-CH_2CH_3$ | thick syrup |
| 1.6 | $-CH_2$(2,4-diCl-Ph) | $-CH_2Cl$ | thick syrup |
| 1.7 | $-CH_2$(2,4-diCl-Ph) | cyclopropyl | thick syrup |
| 1.8 | $-CH_3$ | cyclopropyl | thick syrup |
| 1.9 | $-CH_3$ | $-CH_2CH_3$ | thick syrup |
| 1.10 | $-CH_2$(2,4-diCl-Ph) | -Ph | m.p. 119-120°C |
| 1.11 | $-CH_3$ | -Ph | m.p. 90-91°C |
| 1.12 | $-CH_3$ | $-CH_3$ | thick syrup |
| 1.13 | $-CH_2$(2,4-diCl-Ph) | $-OCH_3$ | thick syrup |
| 1.14 | $-CH_3$ | $-CH_2(CH_2)_2CH_3$ | thick syrup |
| 1.15 | $-CH_2$(2,4-diCl-Ph) | $-N(CH_3)_2$ | thick syrup |
| 1.16 | $-CH(CH_3)$(2,4-diCl-Ph) | $-CH_3$ | thick syrup |
| 1.17 | $-CH(CH_3)$(2,4-diCl-Ph) | $-CH_2CH_3$ | thick syrup |
| 1.18 | $-CH_2CH_3$ | $-CH_3$ | thick syrup |
| 1.19 | $-CH_2CH_2CH_3$ | $-CH_3$ | thick syrup |
| 1.20 | $-CH(CH_3)_2$ | $-CH_3$ | thick syrup |
| 1.21 | $-CH_2(CH_2)_2CH_3$ | $-CH_3$ | thick syrup |
| 1.22 | $-CH_2CH(CH_3)_2$ | $-CH_3$ | thick syrup |

**Table No. 1 continued**

| Cmpd. | $R_1$ | $R_2$ | Characteristic |
|---|---|---|---|
| 1.23 | $-C(CH_3)_3$ | $-CH_3$ | thick syrup |
| 1.24 | -cyclopentyl | $-CH_3$ | thick syrup |
| 1.25 | $-CH_2(CH_2)_6CH_3$ | $-CH_3$ | thick syrup |
| 1.26 | $-CH_2Ph$ | $-CH_3$ | m.p. 98°C |
| 1.27 | $-CH_2CH_3$ | $-CH_2(CH_2)_2CH_3$ | thick syrup |
| 1.28 | $-CH_2CH_2O(2,4\text{-diCl-Ph})$ | $-CH_3$ | thick syrup |
| 1.29 | $-CH_2CH_2OSO_2(4\text{-}CH_3\text{-Ph})$ | $-CH_3$ | thick syrup |
| 1.30 | $-CH_2CH(CH_3)COCH_3$ | $-CH_3$ | thick syrup |
| 1.31 | $-CH_2CH_2OPh$ | $-CH_3$ | thick syrup |
| 1.32 | $-CH_2CH_2(4\text{-}OCH_3\text{-Ph})$ | $-CH_3$ | thick syrup |
| 1.33 | $-CH_2CH_2(4\text{-}OCH_3\text{-Ph})$ | $-CH_2CH_3$ | thick syrup |
| 1.34 | $-CH(CH_3)_2$ | $-CH_2(CH_2)_2CH_3$ | thick syrup |
| 1.35 | $-CH_2Ph$ | $-CH_2(CH_2)_2CH_3$ | thick syrup |
| 1.36 | $-C(CH_3)_3$ | $-CH_2(CH_2)_2CH_3$ | thick syrup |
| 1.37 | cyclopentyl | $-CH_2(CH_2)_2CH_3$ | thick syrup |
| 1.38 | $-CH_2CH(CH_3)_2$ | $-CH_2(CH_2)_2CH_3$ | thick syrup |
| 1.39 | $-CH_2(CH_2)_2CH_3$ | $-CH_2(CH_2)_2CH_3$ | thick syrup |
| 1.40 | $-CH_2(CH_2)_6CH_3$ | $-CH_2(CH_2)_2CH_3$ | thick syrup |
| 1.41 | $-CH_2CH_2CH_3$ | $-CH_2(CH_2)_2CH_3$ | thick syrup |
| 1.42 | $-CH_3$ | $-2\text{-}NO_2\text{-Ph}$ | mp.117-118°C |
| 1.43 | $-CH_3$ | -4-Cl-Ph | thick syrup |
| 1.44 | $-CH_3$ | $-(2\text{-}NO_2\text{-4-Cl-Ph})$ | mp.132-133°C |
| 1.45 | $-CH_3$ | $-4\text{-}OCH_3\text{-Ph}$ | thick syrup |
| 1.46 | $-CH_2(2,4\text{-diCl-Ph})$ | $-CH_2(CH_2)_2CH_3$ | thick syrup |
| 1.47 | $-CH_2(CH_2)_4CH_3$ | $-CH_2(CH_2)_2CH_3$ | thick syrup |
| 1.48 | $-CH_3$ | $-CH_2(CH_2)_3CH_3$ | thick syrup |
| 1.49 | $-CH_3$ | $-CH_2O(2,4\text{-diCl-Ph})$ | mp.93-94°C |
| 1.50 | $-CH(CH_3)(2,4\text{-diCl-Ph})$ | $-CH_2(CH_2)_2CH_3$ | thick syrup |
| 1.51 | $-CH_3$ | -(4-CN-Ph) | thick syrup |
| 1.52 | $-CH_3$ | $-CH_2CH(CH_3)_2$ | thick syrup |
| 1.53 | $-CH_2(CH_2)_3Ph$ | $-CH_3$ | thick syrup |
| 1.54 | $-CH_2(CH_2)_2\text{-}(2\text{-}OCH_3\text{-Ph})$ | $-CH_3$ | thick syrup |

Table No. 1 continued

| Cmpd. | $R_1$ | $R_2$ | Characteristic |
|---|---|---|---|
| 1.55 | $-CH_2CH_2-(4-Cl-Ph)$ | $-CH_3$ | thick syrup |
| 1.56 | $-CH_2CH_2-(4-OCH_3-Ph)$ | $-CH_2(CH_2)_8CH_3$ | thick syrup |
| 1.57 | $-CH_2CH_2-(4-OCH_3-Ph)$ | $-CH_2(CH_2)_2CH_3$ | thick syrup |
| 1.58 | $-CH_2CH_2-(4-OCH_3-Ph)$ | -2,3,4,5,6-penta-F-Ph | thick syrup |
| 1.59 | $-CH_2CH_2-(4-OCH_3-Ph)$ | $-CH=CHCH_3$ | thick syrup |
| 1.60 | $-CH_2CH_2-(4-OCH_3-Ph)$ | $-CH_2Ph$ | thick syrup |
| 1.61 | $-CH_2CH_2-(4-OCH_3-Ph)$ | -cyclopropyl | thick syrup |
| 1.62 | $-CH_2(2,4-(OCH_3)_2-Ph)$ | $-CH_2(CH_2)_2CH_3$ | thick syrup |
| 1.63 | $-CH_3$ | $-(4-NO_2)Ph$ | mp.116-118°C |
| 1.64 | $-CH_3$ | -OPh | thick syrup |
| 1.65 | $-CH_3$ | $-(4-CH_3)Ph$ | thick syrup |
| 1.66 | $-CH_2(2,4-(OCH_3-Ph)$ | $-CH_3$ | thick syrup |
| 1.67 | $-CH_3$ | $-CH_2(CH_2)_5CH_3$ | thick syrup |
| 1.68 | $-CH_3$ | $-CH_2(CH_2)_7CH_3$ | thick syrup |
| 1.69 | $-CH_3$ | $-CH_2(CH_2)_9CH_3$ | thick syrup |
| 1.70 | $-CH_3$ | $-CH=CHCH_3$ (E) | thick syrup |
| 1.71 | $-CH_2(2,4-Cl_2-Ph)$ | $-CH_2(CH_2)_5CH_3$ | thick syrup |
| 1.72 | $-CH_2(2,4-Cl_2-Ph)$ | $-CH_2(CH_2)_9CH_3$ | thick syrup |
| 1.73 | $-CH_2(2,4-Cl_2-Ph)$ | $-CH_2(CH_2)_7CH_3$ | thick syrup |
| 1.74 | $-CH_2CH_2CN$ | $-CH_2(CH_2)_2CH_3$ | thick syrup |
| 1.75 | $-CH_3$ | $-CH=C(CH_3)_2$ | thick syrup |
| 1.76 | $-CH_2CH_2CN$ | $-CH_3$ | thick syrup |
| 1.77 | $-CH_2(3,4-diCl-Ph)$ | $-CH_2(CH_2)_7CH_3$ | thick syrup |
| 1.78 | $-CH_2Ph$ | $-CH_3$ | thick syrup |
| 1.79 | $-CH_2(3,4-diCl-Ph)$ | $-CH_2(CH_2)_5CH_3$ | thick syrup |
| 1.80 | $-CH_3$ | -cyclohexyl | thick syrup |
| 1.81 | -cyclopentyl | $-CH_2(CH_2)_7CH_3$ | thick syrup |
| 1.82 | -cyclopentyl | $-CH_2(CH_2)_5CH_3$ | thick syrup |
| 1.83 | $-CH_2Ph$ | $-CH_2(CH_2)_2CH_3$ | thick syrup |
| 1.84 | $-CH_2Ph$ | $-CH_2(CH_2)_5CH_3$ | thick syrup |
| 1.85 | $-CH_3$ | $-OCH_3$ | m.p. 82-83°C |
| 1.86 | -cyclopentyl | -Ph | thick syrup |

Table No. 1 continued

| Cmpd. | $R_1$ | $R_2$ | Characteristic |
|---|---|---|---|
| 1.87 | $-CH_3$ | $-CH_2CH_2CH_3$ | thick syrup |
| 1.88 | $-CH_3$ | $-C(CH_3)=CHCH_3$ | thick syrup |
| 1.89 | $-CH_2-(4-Cl)Ph$ | $-CH_3$ | m.p. 92-93°C |
| 1.90 | $-CH_3$ | $-CH_2Ph$ | thick syrup |
| 1.91 | $-CH_2-(4-Br)Ph$ | $-CH_3$ | m.p. 73°C |
| 1.92 | $-CH_3$ | $-CH_2(CH_2)_5CH_3$ | thick syrup |
| 1.93 | $-CH_3$ | $-CH_2(CH_2)_4CH_3$ | thick syrup |
| 1.94 | $-CH_2CF_3$ | $-CH_3$ | thick syrup |
| 1.95 | $-C(CH_3)_3$ | $-OCH_3$ | m.p. 87-88°C |
| 1.96 | $-C(CH_3)_3$ | $-CH_2(CH_2)_7CH_3$ | thick syrup |
| 1.97 | $-C(CH_3)_3$ | $-CH=CHCH_3$ | thick syrup |
| 1.98 | -CH(CN)Ph | $-CH_3$ | thick syrup |
| 1.99 | -cyclopentyl | $-OCH_3$ | thick syrup |
| 1.100 | $-CH_3$ | $-O-CH_2-Ph$ | thick syrup |
| 1.101 | -cyclopentyl | $-CH=CH-CH_3$ | thick syrup |
| 1.102 | $-CH_3$ | 2,3,5-tri-I-Ph | m.p. 125-126°C |
| 1.103 | $-CH_2-Ph$ | $-(CH_2)_8-CH_3$ | thick syrup |
| 1.104 | $-CH(CH_3)Ph$ | $-CH_3$ | thick syrup |
| 1.105 | $-CH_2-Ph$ | $-OCH_3$ | thick syrup |
| 1.106 | $-CH_3$ | cyclopentyl | thick syrup |
| 1.107 | $-CH_2-Ph$ | $-CH=CH-CH_3$ | thick syrup |
| 1.108 | $-CH_2$-2-Furyl | $-CH_3$ | thick syrup |
| 1.109 | $-(CH_2)_2-(4-OCH_3-Ph)$ | $-(CH_2)_2-COOCH_3$ | thick syrup |
| 1.110 | $-CH(CH_3)-CH(CH_3)_2$ | $-CH_3$ | thick syrup |
| 1.111 | $-CH_2-CH(CH_3)-CH_2CH_3$ | $-CH_3$ | thick syrup |
| 1.112 | $-CH[CH(CH_3)_2]_2$ | $-CH_3$ | thick syrup |
| 1.113 | $-CH_3$ | $-CH=CH_2$ | thick syrup |
| 1.114 | $-CH_2-C(CH_3)_3$ | $-CH_3$ | thick syrup |
| 1.115 | $-CH_2-CH=CH-CH_3$ | $-CH_3$ | thick syrup |
| 1.116 | $-CH_3$ | 4-F-Ph | m.p. 87-88°C |
| 1.117 | $-(CH_2)_2-OCOCH_3$ | $-CH_3$ | thick syrup |
| 1.118 | $-CH_3$ | 4-Br-Ph | m.p. 96-98°C |

**Table No. 1 continued**

| Cmpd. | $R_1$ | $R_2$ | Characteristic |
|---|---|---|---|
| 1.119 | $-CH_3$ | 4-I-Ph | m.p. 101-102°C |
| 1.120 | $-CH(CH_2COOCH_3)_2$ | $-CH_3$ | thick syrup |
| 1.121 | $-CH_2CH_2-(4-OCH_3-Ph)$ | cyclohexyl | thick syrup |

Example 2-1

Methyl N-[(3,6-dichloro-2-methoxyphenyl)(benzoyloxy)]methylenaminooxyacetate

To a solution of 7.7 g methyl N-(3,6-dichloro-2-methoy)benzoylaminooxyacetate in 250 ml pyridine at -5° C, is added dropwise, a solution of 8.7 ml benzoyl chloride in 10 ml tetrahydrofuran. The resulting mixtures is stirred at 0° C for 1 hr, allowed to warm to ambient temperature, then evaporated in vacuo. The residue is dissolved in ethyl acetate and washed four times with water. After drying with $MgSO_4$, the ethyl acetate solution is evaporated in vacuo to give 14.4 g of crude product as a syrup.

The crude product is purified by silica gel flash chromatography, eluting with 8:2 hexane:tert-butylmethyl ether. Fractions 48-57 are combined and evaporated to yield 3.4 g of a colorless thick syrup that is consistent with the desired product by IR and $^1H$ NMR. The 360MHz $^1H$ NMR spectrum indicates a mixture of stereoisomers consisting of 97% syn-isomer and 3% anti-isomer.

Fractions 80-84 of the chromatography are shown to contain 1.05 g of a solid (m.p. 90-91° C) byproduct of the reaction, corresponding to the N-benzoylated constitutional isomer, as indicatd by the 360MHz $^1H$ NMR spectroscopy. The line broadened spectrum, characteristic of hindered rotation, coalesces at 150° C to a single set of sharp resonances.

In an analogous manner, the following compounds of formula (II) set forth in Tables 2A and 2B are made.

TABLE 2 A

| Compounds of the formula (II), in which X is C(O) | | | |
|---|---|---|---|
| **Cmpd.** | **$R_1$** | **$R_2$** | **Characteristic** |
| 2.2 | $-CH_2$(2,4-diCl-Ph) | -Ph | thick syrup |
| 2.3 | $-CH_3$ | -(2,5-diCl-6-$OCH_3$-Ph) | m.p. 115-116 °C |
| 2.4 | $-CH_3$ | -Ph | thick syrup |
| 2.5 | $-CH_2$(2,4-diCl-Ph) | -(2,5-diCl-6-$OCH_3$-Ph) | m.p. 116-117 °C |
| 2.6 | $-CH_2$(2,4-diCl-Ph) | $-N(CH_3)_2$ | m.p. 130-131 °C |
| 2.7 | $-CH_3$ | -2-$NO_2$-Ph | m.p. 102-103 °C |
| 2.8 | $-CH_3$ | -4-Cl-Ph | m.p. 76-82 °C |
| 2.9 | $-CH_3$ | -4-$OCH_3$-Ph | thick syrup |
| 2.10 | $-CH_3$ | -2-$NO_2$-4-Cl-Ph | thick syrup |
| 2.11 | $-CH_3$ | $-CH_2O$(2,4-diCl-Ph) | m.p. 95-96 °C |
| 2.12 | $-CH_3$ | $-C(CH_3)_3$ | thick syrup |
| 2.13 | $-CH_3$ | -4-CN-Ph | m.p. 99-100 °C |
| 2.14 | $-CH_2CH_2$-(4-$OCH_3$-Ph) | $-CH_2(CH_2)_6CH_3$ | 50 % N-thick syrup |
| 2.15 | $-CH_3$ | -4-$NO_2$-Ph | thick syrup |
| 2.16 | $-CH_3$ | -4-$CH_3$-Ph | thick syrup |
| 2.17 | -cyclopentyl | -Ph | m.p. 87-88 °C |
| 2.18 | $-CH_3$ | $-C(CH_3)=CHCH_3$ | thick syrup |
| 2.19 | $-CH_3$ | -4-F-Ph | m.p. 81-82 °C |
| 2.20 | $-CH_3$ | -4-Br-Ph | m.p. 77-78 °C |
| 2.21 | $-CH_3$ | -4-I-Ph | m.p. 81-82 °C |

TABLE 2B

| Compounds of the formula (II), in which X is $SO_2$. | | | |
|---|---|---|---|
| **Cmpd.** | **$R_1$** | **$R_2$** | **Characteristic** |
| 2.22 | $-CH_2$(2,4-diCl-Ph) | $-CH_3$ | m.p. 102-103 °C |
| 2.23 | $-CH_2$(2,4-$Cl_2$(Ph) | -4-$CH_3$-Ph | m.p. 116-117 °C |
| 2.24 | $-CH_3$ | $-CH_3$ | m.p. 111-113 °C |
| 2.25 | $-CH_3$ | -4-$CH_3$-$Ph_2$ | m.p. 119-120 °C |
| 2.26 | $-CH_2CH_2O$(2,4-diCl-Ph) | $-CH_3$ | thick syrup |
| 2.27 | $-CH_2Ph$ | -Ph | thick syrup |

Example 3-1

Methyl N-(3,6-dichloro-2-methoxy)benzoyl-N-(4-methylphenyl)sulfonylaminooxyacetate

To a solution of 6.2 g methyl N-(3,6-dichloro-2-methoxy)benzoylaminooxyacetate in 200 ml pyridine at -5 °C, is added dropwise, a solution of 11.5 g 4-methylphenylsulfonyl chloride in 10 ml tetrahydrofuran. This mixture is stirred 1 hr at 0 °C, then 17 hrs at room temperature and evaporated in vacuo. The residue is taken up in ethyl acetate and washed four times with water. The organic phase is dried over $MgSO_4$, filtered, and evaporated in vacuo to give 15.7 g crude product as a mixture of N- and O-sulfonylated isomers.

The crude product mixture is purified by flash chromatography on silica gel with 9:1 dichloromethane:hexane. Fractions 6-8 contain 0.7 g of the desired product as a colorless thick syrup. The $^1H$

NMR spectrum, at 360MHz shows the characteristic hindered rotation line-broadening, which coalesces at 150° C to a single set of sharp resonances.

Fractions 20-33 of the initial chromatoraphy contains 1.09 g of a white solid with m.p. 119-120° C which is consistent with the constitutionally isomeric methyl N-[(3,6-dicloro-2-methoxy)phenyl(4-methylphenyl)-sulfonyloxy]methylenaminooxyacetate (syn-isomer) by 360MHz $^1H$ NMR spectroscopy.

In an analogous manner the following compounds of formula I set forth in Table 3 are made.

TABLE 3

| Compounds of formula I in which X is $SO_2$. | | | |
|---|---|---|---|
| **Cmpd.** | **$R_1$** | **$R_2$** | **Characteristic** |
| 3.2 | $-CH_3$ | $-CH_3$-Ph | thick syrup |
| 3.3 | $-CH_2$(2,4-diCl-Ph) | $-CH_3$ | thick syrup |
| 3.4 | $-CH_2$(3,4-diCl-Ph) | $-CH_3$ | 20 % O-subst., thick syrup |
| 3.5 | $-CH_2$(3-Cl-Ph) | $-CH_3$ | 17 % O-subst., thick syrup |
| 3.6 | $-CH_2$(2-$NO_2$-Ph) | $-CH_3$ | 20 % O-subst., thick syrup |
| 3.7 | $-CH_2$(4-F-Ph) | $-CH_3$ | 20 % O-subst., thick syrup |
| 3.8 | $-CH_3$ | $-CH_3$ | thick syrup |

## INTERMEDIATES

Example 4-1

Phenoxyethyl[(3,6-dichloro-2-methoxybenzoyl)aminooxy] acetate

To a solution of 2.15 g of potassium hydroxide in 50 ml of methanol is added 7.55 g of 3,6-dichloro-2-methoxy benzohydroxamic acid followed by stirring until dissolution is obtained. To this solution is added dropwise 3.03 ml phenoxyethylbromoacetate in 25 ml of methanol. The reaction mixture is refluxed for 2 hours and allowed to stir overnight at ambient temperature. To the reaction mixture is added 0.36 g of additional potassium hydroxide and the mixture is heated to reflux for 4 hours, then cooled, filtered, and the filtrate concentrated in vacuo to give a crude semi-solid product. The crude product is taken up in methylene chloride and washed with water, 5% aqueous $NaHCO_3$, brine, then dried over $MgSO_4$, filtered and evaporated in vacuo to give a viscous oil. The is oil crystallized from an ethyl acetate-hexane mixture to the title product.

In an analogous manner, the following compounds of formula (III) set forth in Table 4 are made.

## TABLE 4

Compounds of formula III

| Cmpd. | $R_1$ | Y | Characteristic |
|---|---|---|---|
| 4.2 | $-CH_2(2-Br)Ph$ | O | m.p. 115-116°C |
| 4.3 | $-CH_2(4-Br)Ph$ | O | m.p. 153-155°C |
| 4.4 | $-CH_2(3-Br)Ph$ | O | m.p. 112-115°C |
| 4.5 | $-CH_2[2-CH_3-3,5-di(NO_2)]Ph$ | O | m.p. 110-111°C |
| 4.6 | $-CH_2[2,5-Cl_2-6-OCH_3)Ph$ | O | m.p. 144-145°C |
| 4.7 | $-CH_2[3,4-di(CH_3)-Ph]$ | O | m.p. 134-136°C |
| 4.8 | $-CH_2(2-Cl-6-F-Ph)$ | O | m.p. 154-156°C |
| 4.9 | $-CH_2(3-NO_2-Ph)$ | O | m.p. 75- 77°C |
| 4.10 | $-CH_2(2,5-diCl_2-Ph)$ | O | m.p. 127-129°C |
| 4.11 | $-CH(CH_3)(2,4-diCl-Ph)$ | O | oil |
| 4.12 | $-CH(CH_3)(4-Br-Ph)$ | O | m.p. 128-130°C |
| 4.13 | $-CH_2(4-F-Ph)$ | S | m.p. 114-116°C |
| 4.14 | $-CH_2[3,4,5-tri(OCH_3)-Ph]$ | O | m.p. 118-120°C |
| 4.15 | $-CH(CH_3)Ph$ | O | m.p. 106-108°C |
| 4.16 | $-CH_2[3,4-di(OCH_3)Ph]$ | O | m.p. 125-127°C |
| 4.17 | $-CH_2(3,5-diCl-2-OCH_3-PH)$ | O | m.p. 132-134°C |
| 4.18 | $-CH_2CH_2CN$ | O | m.p. 112-14°C |
| 4.19 | $-CH_2CH_2O(2,4-diCl-Ph)$ | O | m.p. 109-111°C |

Table No. 4 continued

| Cmpd. | $R_1$ | Y | Characteristic |
|---|---|---|---|
| 4.20 | $-CH_2CH_2O(4\text{-}Br\text{-}Ph)$ | O | m.p. 115-118°C |
| 4.21 | $-CH(CH_2CH_3)Ph$ | O | m.p. 93- 94°C |
| 4.22 | $-CH(CH_2CH_2CH_3)Ph$ | O | m.p. 117-118°C |
| 4.23 | $-CH_2COCH_3$ | O | m.p. 101-102°C |
| 4.24 | $-CH_2(CH_2)_2COCH_3$ | O | m.p. 98°C |
| 4.25 | $-CH_2CH_2OCOCH_3$ | O | m.p. 74- 75°C |
| 4.26 | $-CH_2CH_2OSO_2(4\text{-}CH_3)Ph$ | O | m.p. 78- 80°C |
| 4.27 | $-CH_2CH(CH_3)COCH_3$ | O | oil |
| 4.28 | $-CH_2CH_2(4\text{-}OCH_3\text{-}Ph)$ | O | oil |
| 4.29 | $-CH_2C{\equiv}CH$ | O | m.p. 102-103°C |
| 4.30 | $-(CH_2)_3\text{-}(4\text{-}OCH_3\text{-}Ph)$ | O | m.p. 100-101°C |
| 4.31 | $-(CH_2)_4(4\text{-}OCH_3\text{-}Ph)$ | O | oil |
| 4.32 | $-CH_2CH_2(2\text{-}OCH_3\text{-}Ph)$ | O | m.p. 142-144°C |
| 4.33 | $-CH_2[2,4\text{-}di(OCH_3)Ph)$ | O | m.p. 121-122°C |
| 4.34 | $-CH_2(CH_2)_3Ph$ | O | oil |
| 4.35 | $-CH_2CH_2(4\text{-}Cl\text{-}Ph)$ | O | m.p. 98-100°C |
| 4.36 | $-CH_2CH_2O(4\text{-}OCH_3\text{-}Ph)$ | O | m.p. 92- 96°C |
| 4.37 | $-CH_2[3,5\text{-}di(OCH_3)Ph]$ | O | m.p. 100°C |
| 4.38 | $-CH_2CH_2CN$ | O | m.p. 119-120°C |
| 4.39 | $-CH(CN)Ph$ | O | m.p. 124°C |
| 4.40 | $-CH(CH_3)CH(CH_3)_2$ | O | m.p. 124°C |
| 4.41 | $-CH_2CH(CH_3)CH_2CH_3$ | O | m.p. 62- 63°C |
| 4.42 | $-CH[CH(CH_3)_2]_2$ | O | m.p. 114-115°C |
| 4.43 | $-CH_2C(CH_3)_3$ | O | m.p. 118-119°C |
| 4.44 | $-CH(CH_2COOCH_3)_2$ | O | thick syrup |
| 4.45 | $-CH_2CH{=}CHCH_3$ | O | m.p. 71- 73°C |
| 4.46 | $-CH_2CH_2OCOCH_3$ | O | m.p. 79- 80°C |

Biology

The herbicidal activity of the compounds of this application is demonstrated by experiments carried out for the pre-emergence and post-emergence control of a variety of weeds. Such weeds include Abutilon theophrasti, Amaranthus retroflux, Sinapis alba, Solanum nigrum, Bromus tectorum, Setaria viridis, Avena fatua, and Echinochloa crus-galli.

In pre-emergence testing, small plastic greenhouse pots filled with dry soil are seeded with the various weed seeds. Twenty-four hours or less after the seeding, the pots are sprayed with water until the soil is

wet and the test compounds formulated as aqueous emulsions of acetone solutions containing emulsifiers are sprayed at the indicated concentrations emulsifiers are sprayed on the surface of the soil. After spraying, the soil containers are placed in the greenhouse and provided with supplementary heat as required and daily or more frequent watering. The plants are maintained under these conditions for a period of from 14 to 21 days, at which time the conditions of the plants and the degree of injury to the plants is rated.

In post-emergence testing, the compounds to be tested are formulated as aqueous emulsions and sprayed on the foliage of the various weed species that have attained a prescribed size. After spraying, the plants are placed in a greenhouse and watered daily or more frequently. Water is not applied to the foliage of the treated plants. The severity of the injury is determined 21 days after treatment and is rated.

In general, the compounds of this application demonstrate good activity against most of the weed varieties noted above. They are particularly active against Abutilon theophrasti, Amaranthus retroflux, Sinapis alba, Solanum nigrum, and Setaria viridis, in both pre- and post-emergence testing.

Compounds 1.4, 1.8, 1.12, 1.18-1.21, 1.27, 1.33, 1.35, 1.61, 1.65-1.70, 1.75, 1.78, 1.80, 1.83, 1.87, 1.88, 1.93, 1.94, 1.98, 1.100, 1.101, 1.105, 1.106, 1.109, 1.113, 1.116-1.119, 2.16, 2.18, 2.21, 3.5, 3.6, and 3.8 from Tables 1, 2A, and 3 allow substantial control of weeds in greenhouse tests after either or both pre- and post-emergence application at an application rate corresponding to 0.25 kg/ha.

## Claims

1. Compounds of the formulae (I) and (II):

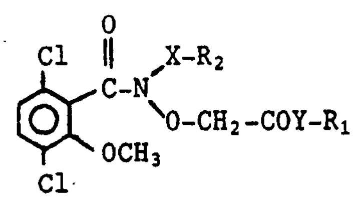

(I)

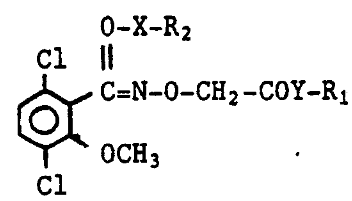

(II)

wherein

X is C(O) or $SO_2$;

Y is O or S;

$R_1$ is selected from the group consisting of H; $C_{1-12}$alkyl, unsubstituted or substituted by halogen; cyano-$C_{1-12}$alkyl; $C_{2-12}$alkenyl; $C_{2-12}$alkynyl; $C_{3-8}$cycloalkyl; $C_{2-5}$alkanoyl-$C_{1-5}$alkyl; $C_{2-5}$alkanoyl-oxy-$C_{1-5}$alkyl; di($C_{1-5}$alkoxy-carbonyl-$C_{1-5}$alkyl)$C_{1-5}$alkyl; Ar-$(SO_2)_{n1}$-$(O)_{n2}$-$C_{1-5}$alkyl, wherein the $C_{1-5}$alkyl moiety is unsubstituted or substituted by CN;

$R_2$ is selected from the group consisting cf H; $C_{1-12}$alkyl; $C_{2-12}$alkenyl; $C_{1-12}$alkoxy; $C_{2-12}$alkynyl; $C_{3-8}$cycloalkyl; benzyloxy; phenoxy; di($C_{1-5}$alkyl)amino; cyano; $C_{1-5}$alkoxy-carhonyl-$C_{1-5}$alkyl; and Ar′-$(O)_{n3}$-$C_{1-5}$alkyl;

Ar and Ar′ are independently phenyl or a five or six membered heteroaromatic ring containing one to three heteroatoms selected from the group consisting of oxygen and nitrogen, which phenyl or hetroaromatic ring may be unsubstituted or substituted by one or more substituents selected from the group consisting of halogen, $C_{1-5}$alkyl, $NO_2$, $C_{1-5}$alkoxy, CN, and $C_{1-5}$alkylthio;

n1, n2, and n3 are independently 0 or 1;

and salt forms thereof.

2. The compound of formula (I) according to claim 1 wherein

$R_1$ is $C_{1-5}$alkyl, unsubstituted or substituted by one to three halogen atoms; $C_{3-6}$cycloalkyl; $C_{1-5}$alkoxy-carbonyl-$C_{1-5}$alkyl; $Ar_1$-$C_{1-2}$alkyl, wherein $Ar_1$ is furyl or is phenyl unsubstituted or substituted by one or

two substituents selected from the group consisting of halogen, nitro and $C_{1-4}$alkoxy; or is Ph-CH(CN), wherein Ph is phenyl; and

$R_2$ is $C_{1-12}$alkyl; $C_{1-4}$alkoxy; $C_{3-6}$cycloalkyl; $C_{2-5}$alkenyl; benzyloxy; or phenyl, monosubstituted by $C_{1-4}$alkyl or halogen.

3. The compound of formula (II) according to claim 1 wherein

X is C(O);

Y is O;

$R_1$ is $C_{1-4}$alkyl; $C_{3-6}$cycloalkyl; and

$R_2$ is $C_{1-4}$alkyl; $C_{2-4}$alkenyl; $C_{3-6}$cycloalkyl; or is phenyl mono-substituted by $C_{1-4}$alkyl or halogen.

4. A compound of formula (IIIa)

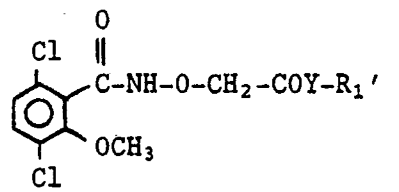

(IIIa)

wherein

Y is O or S; and

$R_1'$ is $C_{1-5}$alkyl substituted by cyano; $C_{2-5}$alkynyl; $C_{2-5}$alkanoyl-$C_{1-5}$alkyl; $C_{2-5}$alkanoyl-oxy-$C_{1-5}$alkyl; $Ar_2$-$C_{1-5}$alkyl wherein the alkyl moiety is substituted by CN; or $Ar_2$-O-$C_{1-5}$alkyl; wherein $Ar_2$ is phenyl, unsubstituted or substituted by one or two substituents selected from halogen and $C_{1-4}$alkoxy.

5. Compounds 4.15, 4.32, 4.33, 4.37, and 4.40, set forth in Table 4.

6. A herbicidal composition comprising a compound of the formula (I) according to claim 1 in free form or in agriculturally acceptable salt form, and an agriculturally acceptable carrier.

7. A herbicidal composition comprising a compound of formula (II) according to claim 1 in free form or in agriculturally acceptable salt form, and an agriculturally acceptable carrier.

8. A herbicidal composition comprising a compound of formula (IIIa) according to claim 4 in free form or in agriculturally acceptable salt form, and an agriculturally acceptable carrier.

9. A herbicidal composition comprising compound 4.15, 4.32, 4.33, 4.37, or 4.40, set forth in Table 4, and an agriculturally acceptable carrier.

10. A process for preparing the compounds of formulae (I) and (II) as defined in claim 1, comprising the step of treating a compound of the formula (III)

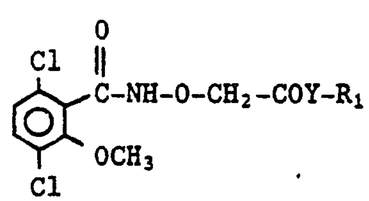

(III)

with an acyl halide or sulfonyl halide of the formula (IV)

V-X-$R_2$ (IV)

wherein X, Y, $R_1$ and $R_2$ are as defined in claim 1, and V is halogen.

11. A process for preparing a compound of the formula (IIIa) comprising the step of reacting a compound of formula (V)

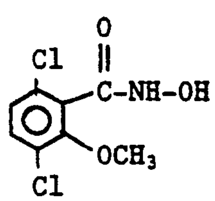

(V)

with a compound of the formula (VI)

$$W\text{-}CH_2\text{-}\overset{\overset{O}{\|}}{C}\text{-}Y\text{-}R_1{}' \quad \text{(VI)}$$

wherein W is halogen, and Y and $R_1{}'$ are as defined in claim 4.

12. A method of combatting weeds comprising applying to the weeds or their locus a herbicidally effective amount of a compound of formulae (I), (II), or (IIIa) as defined in claims 1-4, or compounds 4.15, 4.32, 4.33, 4.37, and 4.40, set forth in Table 4.

13. A process according to claims 10-12, substantially as described herein by way of example.

14. The compounds of formulae (I), (II), (IIIa), as set forth in claims 1-4, whenever obtained by a process according to claims

15. The steps, features, compositions and compounds referred to or indicated in the specification and/or claims of this application, individually or collectively, and any or all combinations or any two or more of said steps or features.

European Patent Office

# EUROPEAN SEARCH REPORT

Application Number

EP 89 81 0934

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 255 800 (SANDOZ)<br>* Entire document *<br>--- | 1-14 | C 07 C 259/10<br>C 07 C 309/68<br>C 07 C 311/48 |
| Y | US-A-3 852 345 (S.B. RICHTER AND L.J. STACH)<br>* Entire document *<br>--- | 1-14 | |
| A | US-A-3 597 467 (S.B. RICHTER AND E.F. BARNAS)<br>* Entire document *<br>--- | 1 | |
| A | FR-A-2 521 130 (VELSICOL)<br>* Entire document *<br>--- | 1 | |
| A | US-A-3 352 899 (K. TANIGUCHI et al.)<br>* Entire document *<br>----- | 1 | |
| | | | TECHNICAL FIELDS SEARCHED (Int. Cl.5) |
| | | | C 07 C 259/00<br>C 07 C 309/00<br>C 07 C 311/00 |

The present search report has been drawn up for all claims

| Place of search | Date of completion of the search | Examiner |
|---|---|---|
| THE HAGUE | 16-03-1990 | WELLS A.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document